# EUROPEAN PATENT SPECIFICATION

(11) **EP 2 233 096 B1**
(45) Date of publication and mention of the grant of the patent: **05.11.2014**
(21) Application number: 08829386.5
(22) Date of filing: 03.09.2008
(51) Int. Cl.: A61B 17/88, A61B 17/17, A61B 17/80

(54) **DEVICE FOR FIXING SCREWS IN OSTEOPOROTIC BONES**
VORRICHTUNG ZUR FIXIERUNG VON SCHRAUBEN IN OSTEOPOROTISCHEN KNOCHEN
DISPOSITIF POUR LA FIXATION DE VIS DANS DES OS OSTÉOPOROTIQUES

(30) Priority: 04.09.2007 ES 200702391; 25.09.2007 ES 200701966 U
(43) Date of publication of application: 29.09.2010
(73) Proprietor: Veme Medico Quirurgica SL, 35012 Las Palmas (ES)
(72) Inventor: GARCES MARTIN, Gerardo, E-35012 Las Palmas (ES); CARTA GONZÁLEZ, Jose Antonio, 35012 las Palmas (ES); YANEZ SANTANA, Alejandro, 35012 las Palmas (ES)
(74) Representative: Carpintero Lopez, Francisco
(86) International application number: PCT/ES2008/000574
(87) International publication number: WO 2009/030794

(56) References cited:
- WO-A1-2005/089660
- CH-A- 537 735
- CH-A5- 653 544
- ES-T3- 2 226 791
- FR-A1- 2 599 962
- FR-A1- 2 599 962
- US-A- 3 867 932
- US-A- 4 502 475
- US-A1- 2004 102 788
- US-A1- 2004 260 291
- US-A1- 2007 088 362

## Description

The present invention relates to a device facilitating the fixing in osteoporotic bones of screws normally used in the osteosynthesis of non-pathological bones. The device in question consists of a biocompatible element and an instrument which is used to handle it, which will act as a fixing nut after being traversed by the screw which has previously passed the bone cortices, thus providing a higher tensile strength of the threaded attachment.

### Background of the Invention

Millions of people suffer from osteoporosis, a typical disease of the elderly, in which the bone loses density. The fundamental consequence is that bone brittleness and, therefore, the possibilities of fractures with even banal traumas, increase. Once the fracture occurs, the use of plates and screws for fixing the bone fragments in a correct alignment, while at the same time the mobilization of the adjacent joints is allowed, is required most of the time. Unfortunately, the loss of bone mass conditions the fixing of the screws being deficient, therefore it is frequent for such screws to give way with the mechanical stressing of the bone in which they are implanted and to be detached from it. The immediate consequence will be the loss of stability in the focal point of the fracture with all the complications that this entails.

In the same way, in bone fractures in young individuals the screws frequently traverse lines or fissures of the bone, in which the fixing is impossible as such screws do not achieve sufficient hold. As in the previous case, the consequence is that the screws are exposed to being easily loosened with the mechanical stressing to which the bone is subjected.

Various systems which attempt to assure the fixing of the screw to the bone have been designed to solve this problem, e.g. FR2599962, from which the preamble of claim 1 is known, or CH653544.

These systems include the use of threaded bars which would traverse the bone and to which a nut would be added on each side; the use of cements which would be added to the perforation orifice before placing the screw such that, upon setting, the fixing of the latter increases; the placement of metal bands and wires which surround the bone and allow fixing the fragments or plates thereto, thus complementing the action of the screws or being used directly without them; the use of expanding materials added to the screws and the use of special screws of various types.

Various types of biocompatible plastics with high tensile strength have been used for years. These plastic can be perforated and threaded without difficulty, assuring a tensile strength even greater than that of a normal bone. Placed against a bone surface they do not create rejection problems and can be a complement for increasing the fixing of a screw to the bone.

The main technical difficulty lies in how to place a fragment of one of these materials so that it is traversed by the screw after the latter has passed the two bone cortices and assure the grip in said fragment. Likewise, a problem which must be solved is the shape and dimensions that said parts must have to prevent them from penetrating the bone cortex due to the pressures generated on such parts.

### Description of the Invention

The device for fixing screws in osteoporotic bones is formed by biocompatible laminar elements assuring the fixing of the screw once the latter has traversed the structure thereof and an instrument facilitating the placement of said elements in the areas of the bone opposite that of the incision, by means of the handling from the side of the cut. The purpose is that said biocompatible material serves for securing the end of the threaded stem of the screw and prevents its loosening. Likewise, the device for fixing screws in osteoporotic bones facilitates the placement of washers or plates on the side of the incision, simultaneously to the fixing of the lamina elements in the face opposite that of the incision.

The instrument used in placing the biocompatible elements for fixing screws in osteoporotic bones is formed by two basic parts, a male part and another female part, connected to one another by a momentum of surface contact, relative linear sliding movement and a degree of freedom. To achieve this type of connection one of the basic parts, referred to as basic male part, has an area in the form of a bar with a prismatic profile which can slide through the orifice which, with a similar profile, the so-called basic female part has made therein.

Each of the basic parts is bent in right angle at one end. When the basic parts are assembled to form the instrument, said bent ends are parallel and form the arms in which the pivoting jaws are assembled, which jaws allow securing and holding the elements of biocompatible material which will be fixed to the bone by means of screws.

Each of said basic parts has a handle. The pair of handles is ergonomically adapted to the palm of a hand to facilitate the action of closing the pivoting jaws.

The base of the handle of the basic female part is perforated according to an axis perpendicular to the longitudinal axis of the handle. The polygonal bar of the male part is coupled in said perforation. Said hollow has a prismatic profile similar to the profile of the bar of the basic male part. The differences lie in the geometric tolerances necessary to allow a suitable play between both parts and in that the perforation has made therein a longitudinal recess with a straight section to allow the passage with clearance of the rack which the prismatic bar has cut in its upper planar surface. The length and thickness of the walls of said perforation are those necessary for unwanted blocks and tilts to not occur between both parts and for them to be able to withstand the mechanical stresses generated during the operation with the instrument.

The arms in which the pivoting jaws are assembled are parallel and are in the same plane as the handles, but the mouth formed by both arms is aimed in a direction opposite to the direction indicated by the mouth formed by the two handles.

In the operation with the instrument, the force exerted by the user with the hand in the handles is that necessary to achieve generating a certain pressure between the pivoting jaws and the bone and to overcome the force of an elastic element which opposes said direction of movement. Said elastic element can be absent.

To prevent the jaws of the instrument from opening by the action of the elastic element when closing forces are no longer exerted in the handles thereof, the instrument has a blocking mechanism. The blocking mechanism is of the catch type. The catch mechanism is formed by a push-button, an elastic blocking element and a rack in the form of saw teeth, which is cut in the upper planar part of the bar with a prismatic profile of the basic male part. The push-button is housed in the handle of the basic female part.

The catch mechanism is of the "normally closed" type, i.e., if the push-button is not acted upon externally, it blocks the opening of the jaws. To achieve that the catch mechanism is of the closed type, it has an elastic blocking element in the handle of the basic female part.

The blocking push-button is formed by a rod which in its lower part has a tooth which may be embedded in the rack of the basic male part to block the opening of the jaws of the instrument and in its upper part it has means so that, by means of the pressure of a finger, the force of the elastic blocking element can be overcome and the push-button is disconnected from the rack, whereby the opening of the jaws is unblocked.

When the instrument does not contain the mentioned elastic element, the catch mechanism and the blocking push-button will not be necessary. To block the two parts in a certain position (normally when both jaws brace the bone), the female part will have a threaded drill hole made in one of the side faces of the base of the handle, through which the polygonal bar of the male part slides. Said threaded perforation will allow, by means of using a pressure bolt with a suitable head to facilitate the manual screwing, blocking or unblocking the relative movement between the male and female parts, upon manually applying a small tightening torque in the head of said bolt.

The limitation of the degree of opening of the jaws of the instrument is fixed by a closure element with a form or with a closure by friction, located at the end of the prismatic bar of the basic male part.

The pivoting jaw corresponding to the basic male part has a rectangular parallelepiped shape. This element has cut therein a hollow in the face which will be in contact with the bone. Said hollow has the suitable shape to house and transport the biocompatible element which is to be fixed and to facilitate the extraction of the instrument once the biocompatible element has been fixed to the bone. The remaining surface of said face which is not occupied by the hollow is knurled to achieve a suitable roughness and to prevent it from sliding once it has pressed against the bone.

The upper and lower faces of this pivoting jaw have drilled therein a blind cylindrical hole in their geometric center and perpendicularly thereto, in which the elements performing the function of shaft of the articulation are introduced.

The pivoting jaw assembled in the basic female part is in the form a polyhedron with six faces. Two opposite faces of said polyhedron are not planar but concave and are those which will be in contact with the bone or with the element which will be placed in the bone. Said curved faces differ from one another by the geometry of the hollow which they have made in their surface. The selection of the face the normal of which is aimed at the bone depends on the type of element which is to be fixed in the area of the incision. In other words, if it is an osteosynthesis plate or washer. The face designed to place washers has a circular hollow with the suitable depth so that same can be correctly housed. The face designed to place osteosynthesis plates has small flanges which will be introduced in the oval holes thereof, facilitating the positioning of the instrument and preventing the unwanted slides thereof with respect to the plates.

The upper and lower faces of this pivoting jaw have a blind cylindrical hole made in their geometric center. Said drill hole is perpendicular to said faces and is where the elements carrying out the function of shaft of the articulation are introduced.

To allow the passage of the bits which must make holes in and the biocompatible securing elements requiring it, as well as the advance of the screws which are inserted, the pivoting jaws have through hollows perpendicular to the faces which will be in contact with the bone.

The arm in which the pivoting jaw corresponding to the basic female part is assembled, once the instrument has been assembled, is perpendicular to the prismatic bar of the basic male part. The arm in which the pivoting jaw corresponding to the basic male part is assembled is perpendicular to the longitudinal axis of its prismatic bar. Therefore, the two arms in which the pivoting jaws are assembled are parallel. Each of the two arms in which the pivoting jaws are assembled has two flat bars perpendicular thereto and parallel to one another. The flat bars of an arm will face those of the other arm, but their longitudinal axes do not have to coincide. Said flat bars are cantilevered and serve as support for their respective pivoting jaw. The flat bars have threaded holes in which the elements which must serve as rotation shaft of the pivoting jaw are connected. The pivoting jaw installed in the basic female part can rotate 360° with respect to its rotation shaft without setbacks, this feature allowing choosing the face of the pivoting jaw which must face the bone; said choice will depend on the type of element which is to be placed, i.e., if it is an osteosynthesis plate or washer.

The arm in which the pivoting jaw of the basic female part is assembled has an element designed to allow the passage of the screws which must be inserted in the bone and to easily couple different suitable bit guides to the bit diameter which is to be used at any time.

The biocompatible fixing parts are laminar-shaped elements of biocompatible plastic material which are placed in the areas of the bone opposite that of the incision by means of the handling from the side of the cut. Said elements are placed in the instrument, in the face of the jaw of the male part facing the jaw of the female part. Thus, when the jaw of the male part contacts the bone, it also puts into contact the fixing part therewith so that the bit or the screw which have previously traversed the jaw of the female part and the two cortices of the bone directly penetrate the biocompatible fixing part. The purpose is that said biocompatible material serves for securing the end of the threaded stem of the screw and prevents its loosening.

The biocompatible fixing part can have made in its center a perforation with a diameter less than the smaller diameter of the thread of the osteosynthesis screws so that the latter can advance when turned, while creating their own thread, in said perforation or be introduced after making a thread with the suitable instruments.

As a result of the mechanical characteristics of biocompatible plastic materials, which allow a relatively easy self-tapping of the osteosynthesis screws, it is not necessary for the latter, when they are introduced from the area of incision and are aimed towards the opposite area of the bone in which the fixing element of the present invention waits, to have their axis perfectly aligned with the axis of the perforation of the securing element, but rather they can be inserted with a certain angle of incidence with respect to the axis of the perforation, and therefore with respect to the normal to the bone, maintaining a sufficient block against the loosening of the attachment.

The fixing element of the invention, which has a laminar shape, can be placed in planar bones or bones with certain convexity, therefore, the surface which will be in contact with the bone cortex may be planar or have a concave shape for the purpose of adapting as best as possible to the cortical surface of the bone. The surface which will be covered by the tissues will have the suitable shape and finish for a better adaptation thereof and to prevent them from being damaged.

Upon applying tightening torques to the osteosynthesis screws in the placement thereof or when the bone is subjected to mechanical stressing, tensile stresses will occur in the stem of the screw which will generate pressures between the bone cortex and the surface of the securing element which is in close contact therewith. In order for the securing element model of this invention to not penetrate the bone cortices as a consequence of these pressures, it will have contact surface dimensions suited to each circumstance.

The thicknesses of the securing elements will depend on the thread pitches of the osteosynthesis screws which are used and on the mechanical strengths required.

### Advantages of the Invention

The present invention has the advantage over others previously described that the threading inside the biocompatible element assuring the fixing of the screw is carried out at the time of introducing same. Since it is previously drilled and is preferably made of plastic material, the use of a threading prior to introducing the screw will generally not be necessary, given that the thread will be created by the advance of the screw itself inside the fixing element. This will allow the use of any type of screw in all the fixing elements, which considerably reduces the cost of the manufacturing and use process.

The instrument used to place the fixing element is essential for the introduction of the screw therein. Given that the advance end of the screw is normally visible for the surgeon, the placement of the fixing element would be very complicated if performed manually. The instrument of the present invention has jaws and a system of guides assuring that the screw introduced by the jaw of the female part always finds the drill hole of the element placed in the jaw of the male part. When the screw is introduced inside the fixing element, the latter is easily released from the jaw containing it and is pressed against the bone, assuring the stability of the screw inside the bone, upon acting as a locking nut.

### Brief Description of the Drawings

In order to better understand what is described in this specification, several drawings are attached in which, only by way of example, a practical case of preferred embodiment of the device for fixing screws in osteoporotic bones is set forth.

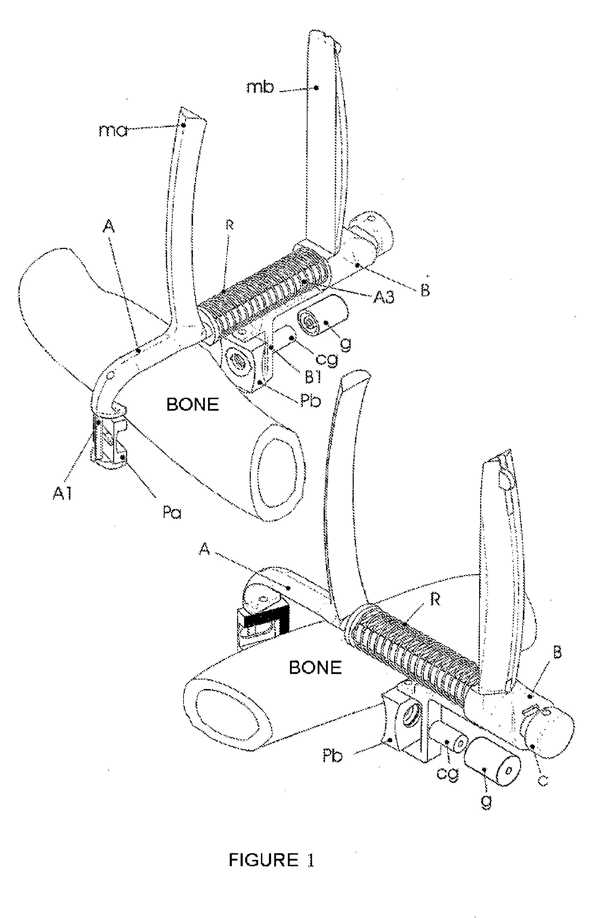

Figure 1 are two isometric perspective views of the assembly of the tool which used as an aid for understanding some of the claims. It shows a sketch of the bone, the basic male part (A), the basic female part (B), the handle of the basic male part (ma), the handle of the basic female part (mb), the clamp-opening spring (R), the pivoting jaw of the basic male part (Pa), the pivoting jaw of the basic female part (Pb), the right angle arm (A1) of the basic male part (A) in which the pivoting jaw (Pa) is assembled, the right angle arm (B1) of the basic female part (B) in which the pivoting jaw (Pb) is assembled and the interchangeable bit guides (g) are connected, and the prismatic bar (A3) of the male part (A) in which the graduated ruler is located.

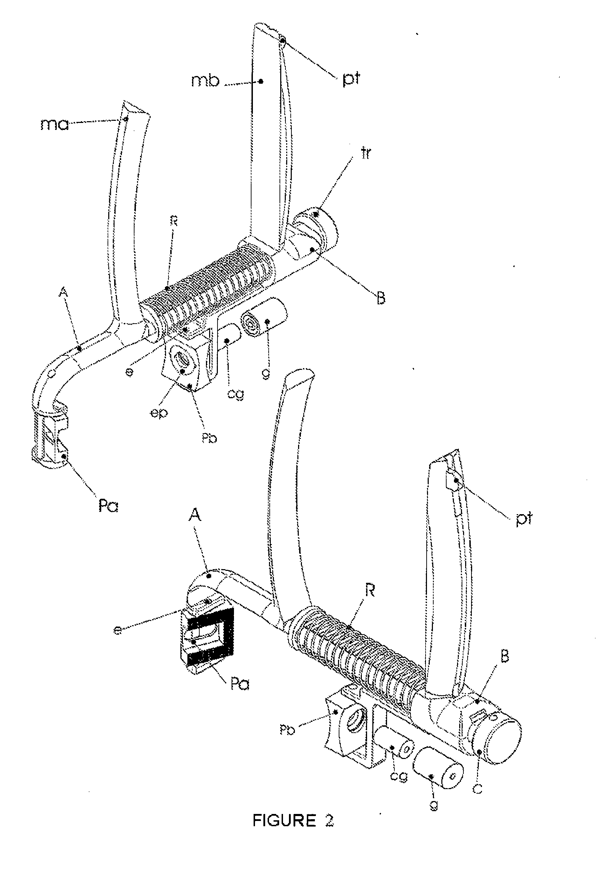

Figure 2 are two isometric perspective views of the assembly of the tool. It shows the basic male part (A), the basic female part (B), the handle of the basic male part (ma), the handle of the basic female part (mb), the clamp-opening spring (R), the pivoting jaw of the basic male part (Pa), the pivoting jaw of the basic female part (Pb), the head of the push-button of the catch mechanism (pt), the interchangeable bit guide connector (cg), a bit guide (g), the closure element (C) which limits the opening of the clamp, closure element-retaining screw (tr), rotation shaft of the pivoting jaw (e), and the element with flanges to be secured to plates (ep).

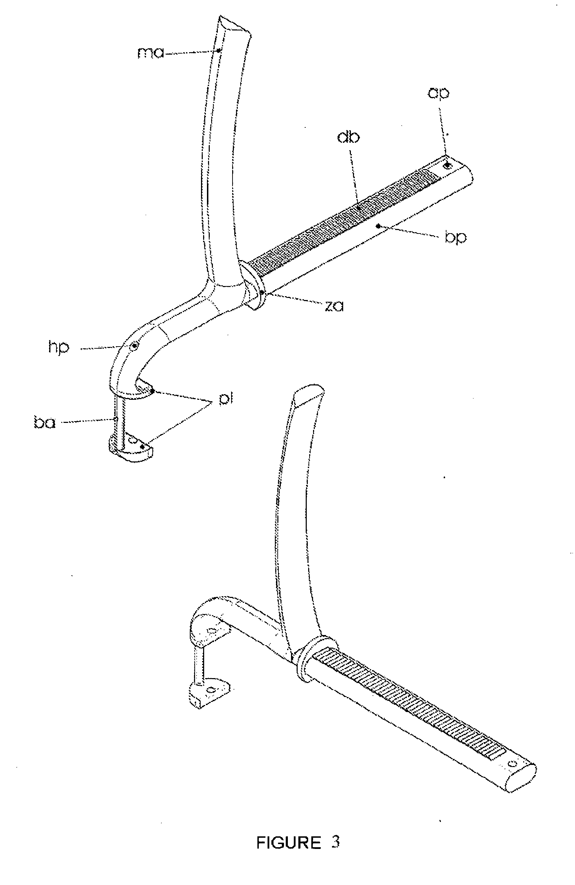

Figure 3 are two isometric perspective views of the basic male part. It shows the handle (ma), the prismatic bar (bp) with the upper surface cut in a saw-toothed manner (db), the area for supporting the end of the jaw-opening spring (za), the arm for assembling the pivoting jaw of the basic male part (ba), with the two flat bars (pl), the housing of the tip of the setscrew (ap) and the through hole (hp) for assembling the upper rotation shaft of the pivoting jaw.

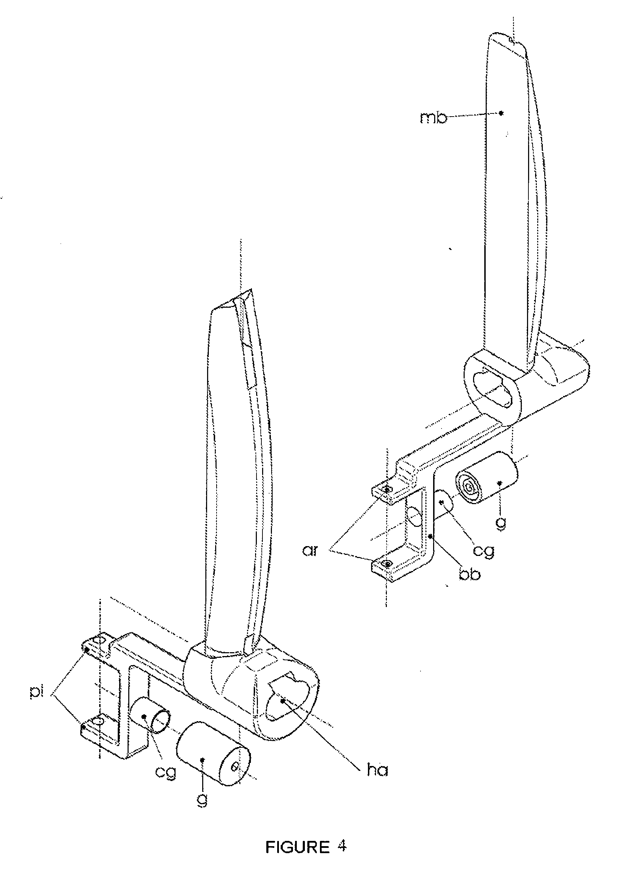

Figure 4 are two isometric perspective views of the basic female part. It shows the handle (mb), the arm (bb) for assembling the pivoting jaw of the basic female part, with the two flat bars (pl), with the threaded holes (ar) for installing the rotation shafts of the pivoting jaw, the housing (ha) of the prismatic bar of the basic male part, the bit guide connector (cg) and an interchangeable bit guide (g).

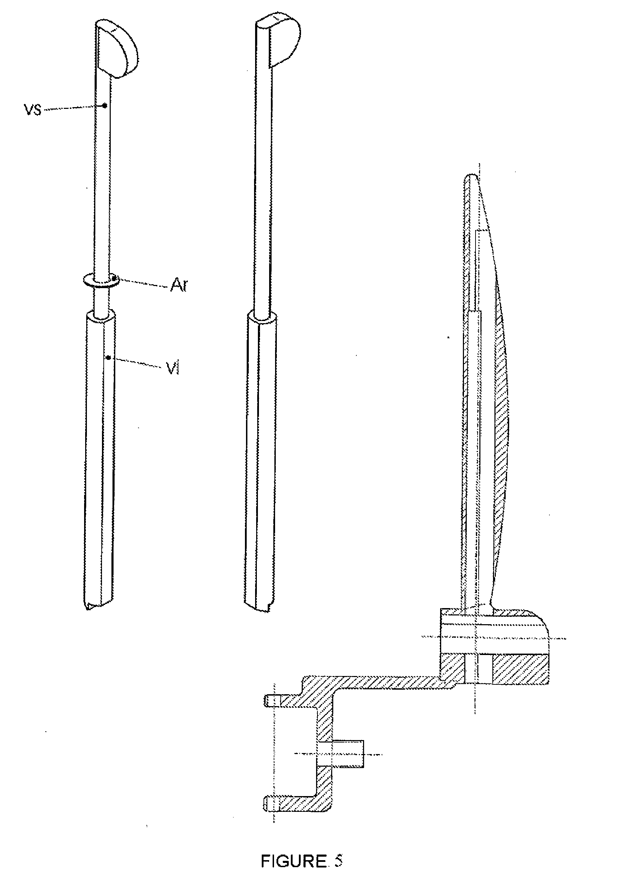

Figure 5 shows two isometric perspective views of the push-buttons of the catch mechanism and a section of the basic female part. In Figure 5 the two rods (vs and vi) forming the push-button are assembled, therefore the threaded attachment coupling them is not observed. However, the washer (Ar) which must be assembled to serve as a support for the spring (not depicted) of the catch mechanism, has been depicted in one of the views. In the section of the basic female part the hollow for assembling and housing the push-button can be observed.

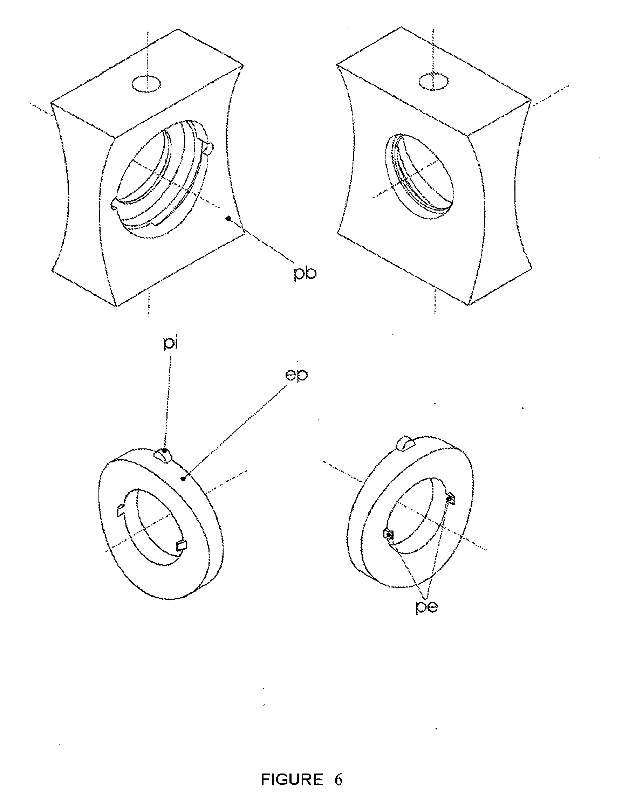

Figure 6 shows two isometric perspective views of the pivoting jaw (pb) corresponding to the basic female part. Likewise, two isometric perspective views of the part (ep) with flanges which would be used in the preferred manufacture are shown. In the part with flanges, the actual flanges (pe) and one of the tabs (pi) designed for the bayonet assembly of the part can be observed.

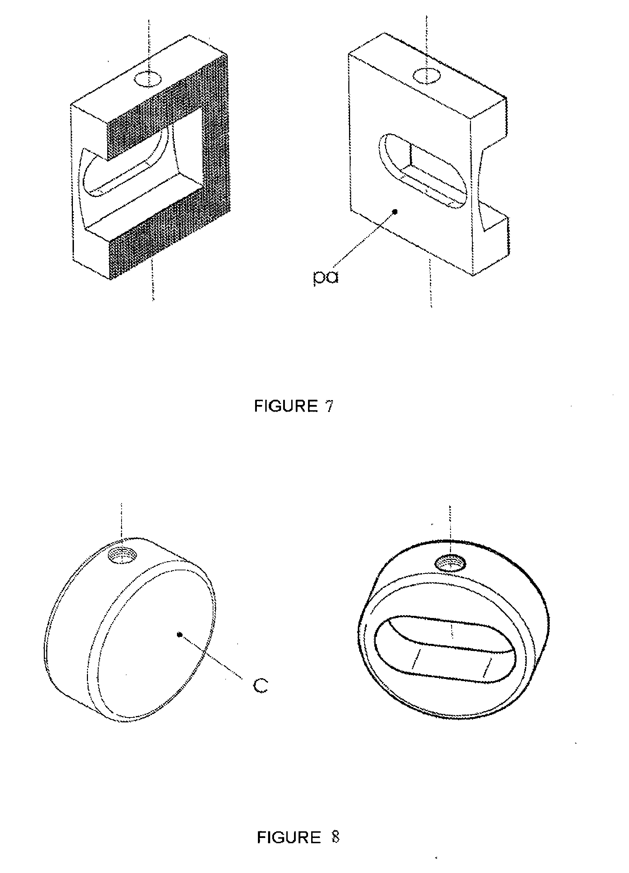

Figure 7 shows two isometric perspective views of the pivoting jaw (pa) corresponding to the basic male part. The housing for inserting the biocompatible element which must be fixed to the bone is rectangular parallelepiped in this case, but it can have any geometric shape.
Figure 8 shows two isometric perspective views of the closure element (C) limiting the opening of the clamp. The threaded hole for installing the retaining screw and the blind hollow for inserting the end of the prismatic bar of the basic male part can be observed.

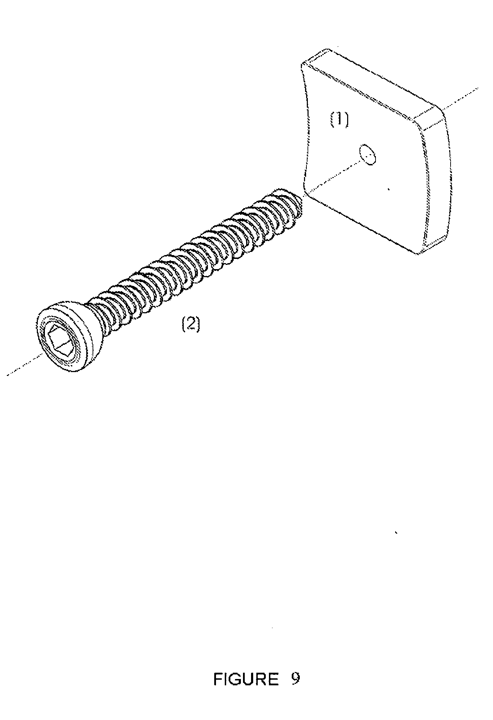

Figure 9 shows a perspective view of a fixing element for osteosynthesis screws for osteoporotic bones, previously perforated (1) (preferred embodiment), and a typical osteosynthesis screw (2).

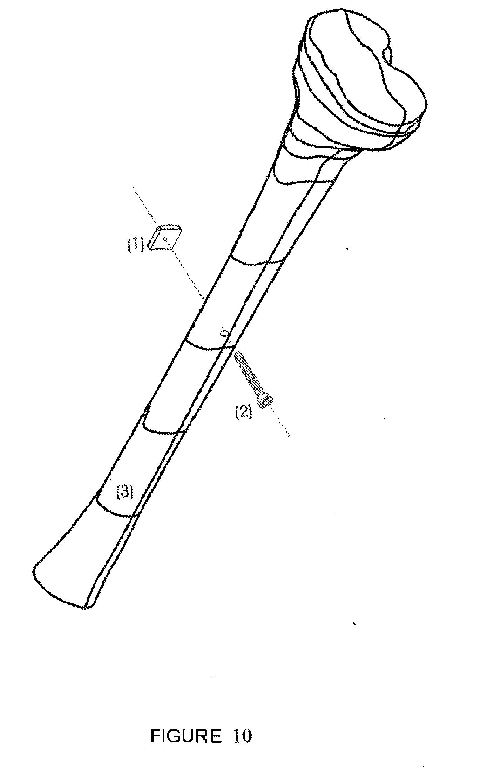

Figure 10 shows a perspective view of a fixing element (1) for osteosynthesis screws, a long cylindrical type bone (3) and a typical osteosynthesis screw (2), for the purpose of detailing one of the uses of said element.

### Description of a Preferred Embodiment

The device for fixing screws in osteoporotic bones is formed by biocompatible fixing elements and an instrument consisting of two basic (male and female) parts connected to one another by a pair of surface contact, relative linear sliding movement and a degree of freedom. To achieve this type of connection one of the basic parts, referred to as basic male part, has an area in the form of a bar with a prismatic profile which can slide through the orifice which, with a similar profile, the so-called basic female part has made therein. Said profile is circular in its sides and planar in the upper and lower parts. With this type of profile the relative rotation of a basic part with respect to the other one is prevented and the relative axial translation movement thereof is facilitated.

Each of the basic parts has an arm for assembling a pivoting jaw. When the basic parts are assembled to form the instrument, said arms are parallel and allow securing, as a result of the pivoting jaws, the elements of biocompatible material which will be fixed to the bone by means of screws.

Each of said basic parts has a handle. The pair of handles is ergonomically adapted to the palm of a hand to facilitate the action of closing the jaws.

The base of the handle of the female part is perforated according to an axis perpendicular to the longitudinal axis of the handle. The prismatic bar of the male part is coupled in said perforation. Said hollow has a prismatic profile similar to the profile of the bar of the basic male part. The differences lie in the geometric tolerances necessary to allow a suitable play between both parts and in that the perforation has made therein a longitudinal recess with a straight section to allow the passage with clearance of the rack which the prismatic bar has cut in its upper surface. The length and thickness of the walls of said perforation are those necessary for unwanted blocks and tilts to not occur between both parts and for them to be able to withstand the mechanical stresses generated during the operation with the instrument.

The arms in which the pivoting jaws are assembled are in the same plane as the handles, but the mouth formed by the two arms has a direction opposite to the direction of the mouth formed by the two handles. Each of the arms has pivoting jaws which transport and secure the biocompatible elements which must be fixed to the bone by means of screws.

In the operation with the instrument, the force exerted by the user with the hand in the handles is that necessary to achieve generating a certain pressure between the two jaws and the bone and to overcome the force of a compression coil spring with a circular section which opposes said direction of movement. This compression coil spring is longitudinally traversed by the bar with a prismatic profile of the basic male part, which, in addition to serving as a guide for the latter, prevents the buckling tending to occur therein due to its fineness. The right angle ends of this spring are supported in the bases of the handles, which are conditioned to serve as a seat for them.

To prevent the jaws of the instrument from opening by the action of the elastic element when closing forces are no longer exerted in the handles thereof, the instrument has a blocking mechanism. The blocking mechanism is of the catch type. The catch mechanism is formed by a push-button, a blocking spring and a rack in the form of saw teeth, which is cut in the upper planar part of the bar with a prismatic profile of the basic male part.

The push-button and the blocking spring are housed in the perforation made in the longitudinal axis of the handle of the basic female part.

The catch mechanism is of the "normally closed" type, i.e., if the push-button is not acted upon externally, it blocks the opening of the jaws. To achieve that the catch mechanism is of the closed type, it has a compression coil spring with right angle ends.

The blocking push-button is formed by two rods with constant sections which are connected by means of a threaded attachment. The lower rod has a prismatic section with two axes of symmetry. Two sides of this section are straight and parallel and the other two sides are circular with a center in the center of the profile. The lower end of this rod has a projection in the form of a saw tooth which is inserted in the rack of the basic male part to block the opening of the clamp of the instrument. The upper rod has a circular section and its diameter is less than the perpendicular distance existing between the two straight and parallel sides of the section of the lower rod. The upper end of this rod will have a side projection, which will be ergonomically adapted to the thumb to be comfortably pushed by the latter when the push-button is to be disconnected from the rack and the opening of the jaws is to be unblocked.

The blocking spring supports one of its ends in the step existing between the two rods forming the push-button. The other end of the spring is seated in a cylindrical washer which is located in a step, generated by a change of section, which the longitudinal perforation of the handle of the basic female part has.

The axial perforation of the handle of the basic female part consists of two segments separated by a step. The lower segment, which is the greater part of the total length of the tunnel, has a prismatic section with a shape similar to the lower rod of the push-button. The upper segment has a circular section with the suitable dimensions to allow the passage with play of the upper rod of the push-button. In order to allow, at the time of the assembly, the side projection of the upper segment of the push-button to travel over the axial perforation of the handle of the basic female part, the latter will have a groove with a rectangular section made in its entire length.

The limitation of the degree of opening of the jaws of the instrument is fixed by a closure element located at the end of the prismatic bar of the basic male part. Said closure element has an outer cylindrical shape and has a blind perforation with a prismatic section to be adjusted to the shape of the end of the bar. Once the compression spring and the basic female part, with its corresponding catch mechanism, have been inserted in the prismatic bar of the basic male part, the closure element is assembled. The fixing of the closure element to the bar of the basic male part is carried out by means of a coupled-type retaining screw which is screwed perpendicularly to the side surface of the closure element. The coupled screw with a conical end assured a positive fixing, since the end of the screw enters an orifice drilled in the prismatic bar of the basic male part.

To measure the degree of opening of the jaws, the prismatic bar of the basic male part has in its sides a graduated scale and the basic female part serves as a signaling slide.

The pivoting jaw assembled in the basic male part has a rectangular parallelepiped shape. This element has cut therein a hollow in the base which will be in contact with the bone. Said hollow has the suitable shape to house and transport the biocompatible element which is to be fixed and to allow extracting the instrument once the biocompatible elements have been fixed to the bone. The remaining surface of said base which is not occupied by the hollow is knurled to achieve a suitable roughness and to prevent it from sliding once it has pressed against the bone.

The upper and lower faces of this pivoting jaw have drilled therein a blind cylindrical hole in their geometric center and perpendicularly thereto, in which the pins performing the function of pivot element of the articulation are introduced.

The pivoting jaw corresponding to the basic female part is in the form of a polyhedron with six faces. Two opposite faces of said polyhedron are not planar but concave and are those which will be in contact with the bone or will face it. Said curved faces differ from one another by the geometry of the hollow which they have made in their surface and by the surface roughness thereof. The selection of the face which will be in contact with the bone depends on the type of element which is to be placed in the area of the incision. In other words, if it is an osteosynthesis plate or washer. The face designed to place washers is rough and has a cylindrical hollow with the suitable depth so that same can be correctly housed. In the preferred configuration, the face designed to place plates has a cylindrical hollow in which a cylindrical part may be assembled and disassembled, which cylindrical part has a central oval through hole and two small flanges in the end edges of said hole. Said flanges will be introduced in the oval holes of the plates, facilitating the positioning of the instrument and preventing the unwanted slides thereof with respect to the plates. The assembly of the element provided with flanges in the pivoting jaw is carried out by means of a bayonet connection, also called bayonet closure or bayonet assembly, which forms a type of quick fixing and coupling between the inter-corresponding surfaces of the pivoting jaw and the part with flanges. To couple the two surfaces it is necessary to align and fit the two tabs or side protrusions of the element with flanges with the notches made in the hollow of the pivoting jaw. Once the tabs reach the run or stop of the notches, the element with flanges is rotated with respect to the pivoting jaw such that the protrusions are guided towards perpendicular notches arranged to prevent their misalignment. Optionally, a spring can be used to maintain the securing force.

The upper and lower faces of this pivoting jaw have drilled therein a blind cylindrical hole in their geometric center and perpendicularly thereto, in which the pins performing the function of pivot element of the articulation are introduced.

To allow the passage of the bits which must make holes in and the biocompatible securing elements requiring it, as well as the advance of the screws which are inserted, the pivoting jaws have through hollows perpendicular to the faces which will be in contact with the bone.

The arm of the basic female part in which its corresponding pivoting jaw is assembled arises perpendicular to the end of the extension which, in the form of a bar with a rectangular section, its handle has in its lower segment. The longitudinal axis of said extension is parallel to the longitudinal axis of the prismatic bar of the male part. This arm of the female part has two cantilevered parallel flat bars serving as a support for one of the pivoting jaws. To that end, these flat bars have threaded holes in which the elements which must serve as rotation shaft of the pivoting jaw are connected. These elements are a hybrid between an articulation bolt and a setscrew. The length of the flat bars and the position of the threaded holes are such that they allow a 360° rotation of the pivoting jaw. Likewise, this arm of the basic female part has a cylindrical element and a hollow with a longitudinal axis perpendicular to said arm and which has been designed to couple bit guides with different diameters and to allow the passage of the screws which must be inserted in the bone. The element for coupling bit guides has elements for blocking the guides.

The elements forming the rotation shafts of the pivoting jaws are rods threaded at one of the ends. The threaded area is connected with the female thread machined in the holes of the flat bars. The non-threaded segment is smooth and is introduced with play in the blind holes which the pivoting jaws have made in their upper and lower faces. To perform the assembly and disassembly, these shaft elements have an inner Allen type hexagon at the threaded end.

The arm of the basic male part in which the corresponding pivoting jaw is assembled forms ninety degrees with the longitudinal axis of its prismatic bar. Said arm is the one which will be introduced between the tissues and the bone in order to install, by means of the aid of its pivoting jaw, the biocompatible elements which must be placed in the face opposite that of insertion of the bits and screws to act as fixing nuts for the latter. Like the arm of the basic female part, the arm of the basic male part in which the pivoting jaw is installed has two cantilevered parallel flat bars serving as a support for same. However, the length of these flat bars is smaller, since a complete rotation of this pivoting jaw with respect to its rotation shaft is not required. The shaft elements of this pivoting jaw are identical to those used in the basic female part.

In the operation with the instrument, the pivoting jaws, by means of pressure, maintain the elements of biocompatible material in contact with the bone. As a result of the freedom of each pivoting jaw to rotate in its corresponding arm with respect to an axis parallel to the longitudinal axis of the handles, the longitudinal axis of the male part can be located in a range of angular positions with respect to the longitudinal axis of the bone. Therefore, with the instrument it is possible to drill holes and to screw screws which form different angles with respect to the longitudinal axis of the bone.

The preferred embodiment of the biocompatible fixing element is that of a laminar-shaped securing element of biocompatible plastic material with an concave inner surface according to an axis and an outer convex surface according to the same axis, which is suitable to be placed in cylindrical type bones such as femurs, humeris, etc., and which has made therein a cylindrical perforation with an axis perpendicular to the surface which will be in contact with the bone cortex and with a diameter less than the smaller diameter or root diameter of the thread of the osteosynthesis screw which must be used in the fixing. In the preferred embodiment, the projection of the securing element has a square shape. The radius of curvature of the surface of contact with the bone is similar to that of the surface of the bone in which it is placed and the thickness of the lamina is between 2 mm and 5 mm.

## Claims

1. Device for fixing a screw in an osteoporotic bone comprising:
- a biocompatible fixing element (1) and an instrument facilitating the placement therefor, the instrument comprising:
- a male part (A) and a female part (B), the male part (A) and the female part (B) having a surface contact between them and a degree of freedom of relative movement; the male part (A) and the female part (B) comprising handles (ma,mb), and the male part (A) and the female part (B) being adapted to slide linearly with respect to one another;
- a first jaw (Pa) assembled in a first right angle arm (A1) forming an end of the male part (A) to be moved, at will, the first jaw (Pa) being adapted to house and transport the biocompatible fixing element (1),
- a second jaw (Pb) assembled in a second right angle arm (B1) forming an end of the female part (B);
- and a guide element (g), connected to the second right angle arm (B1) by means of a connector (cg), a screw being introducable through the guide element (g);
**characterized in that** the second jaw (Pb) is a pivoting jaw adapted to rotate 360° with respect to a pivot axis, and enables to select between:
- a first face of the second jaw (Pb) comprising a rough surface and a cylindrical hollow adapted to press an osteosynthesis washer against the bone; and
- a second face of the second jaw (Pb) comprising a hollow with flanges in the edges adapted to press an osteosynthesis plate against the bone.

2. Device for fixing screws in osteoporotic bones according to the previous claim, **characterized by** comprising a block mechanism, the block mechanism comprising a push-button, an elastic blocking element and a rack shaped as saw teeth.

3. Device for fixing screws in osteoporotic bones according to any of the previous claims, **characterized in that** the first jaw (Pa) is a pivoting jaw adapted to rotate with respect to a pivot axis, enabling to select between:
- a first face of the first jaw (Pa) comprising a hollow with a geometric shape adapted for housing the biocompatible fixing element;
- and a second face of the first jaw (Pa), the second face of the first jaw (Pa) being knurled.

4. Device for fixing screws in osteoporotic bones according to any of the previous claims, **characterized in that** the second face of the second jaw (Pb) comprises a cylindrical part with a central oval through-hole and two flanges in the end edges of the through-hole.

5. Device for fixing screws in osteoporotic bones according to any of the previous claims, **characterized in that** the male part (A) comprises a prismatic bar (A3) with graduated scales and **in that** the female part (B) comprises a slider, the graduated scales and the slider being adapted to measure the opening of the first jaw (Pa) and the second jaw (Pb).

6. Device for fixing screws in osteoporotic bones according to the previous claims, **characterized in that** longitudinal axes of the handles (ma,mb) are in a same vertical plane as longitudinal axes of the first arm (A1) and the second arm (B1); and **in that** the handles (ma,mb) form a mouth aimed in an opposite direction as a mouth formed by the first arm (A1) and the second arm (B1).

7. Device for fixing screws in osteoporotic bones according to claim 5 or claim 6 when dependent on claim 5, **characterized by** comprising a closure element (C) in an end of the prismatic bar (A3) of the male part (A), the closure element (C) being adapted to limit a degree of opening of the first jaw (Pa) and the second jaw (Pb), and the closure element (C) comprising:
- an outer cylindrical shape;
- a blind perforation with a prismatic section adapted to be adjusted to a shape of the end of the prismatic bar;
- and a coupled-type retaining screw with a conical end, adapted to be screwed perpendicularly to a side surface of the closure element (C) and to enter an orifice drilled in the prismatic bar.

8. Device for fixing screws in osteoporotic bones according to any of the previous claims, **characterized in that** the connector (cg) is adapted to couple interchangeable bit guides (g) with a plurality of diameters.

9. Device for fixing screws in osteoporotic bones according to claim 5 or to one of the previous claims when dependent on claim 5, **characterized by** comprising a compression spring (R) assembled coaxially with the prismatic bar (A3) of the male part (A), adapted to maintain the handles (ma, mb) of the male part (A) and female part (B) separated.

10. Device for fixing screws in osteoporotic bones according to any of the previous claims, **characterized by** comprising a pressure bolt adapted to block and unblock a relative movement between the male part (A) and the female part (B), the pressure bolt comprising a head adapted to screw the pressure bolt by manually applying a tightening torque.

11. Device for fixing screws in osteoporotic bones according to any of the previous claims, **characterized in that** the biocompatible fixing element is a laminar-shaped fixing element with a perforation, the perforation having a diameter smaller than a thread of the screw.

12. Device for fixing screws in osteoporotic bones according to any of the previous claims, **characterized in that** surfaces of the biocompatible fixing element are adapted to characteristics of a bone cortex in which the fixing elements are adapted to be placed.

## Patentansprüche

1. Vorrichtung zur Fixierung einer Schraube in einem osteoporotischen Knochen, mit:
einem biokompatiblen Fixierelement (1) und einem Instrument, das dessen Platzierung erleichtert, wobei das Instrument aufweist:
einen männlichen Teil (A) und einen weiblichen Teil (B), wobei sich der männliche Teil (A) und der weibliche Teil (B) in gegenseitigem Oberflächenkontakt befinden und einen Freiheitsgrad zur Relativbewegung aufweisen, wobei der männliche Teil (A) und der weibliche Teil (B) Griffe (ma, mb) aufweisen und der männliche Teil (A) und der weibliche Teil (B) relativ zueinander linear gleitbar sind;
einer ersten Klaue (Pa), die an einem ersten rechtwinkligen Arm (A1) angebracht ist, der ein beliebig bewegbares Ende des männlichen Teils (A) bildet, wobei die erste Klaue (Pa) in der Lage ist, das biokompatible Fixierelement (1) aufzunehmen und zu transportieren;
einer zweiten Klaue (Pb), die an einem zweiten rechtwinkligen Arm (B1) angebracht ist, der ein Ende des weiblichen Teils (B) bildet;
und einem Führungselement (g), das mittels eines Konnektors (cg) mit dem zweiten rechtwinkligen Arm (B1) verbunden ist, wobei eine Schraube durch das Führungselement (g) einführbar ist;
**dadurch gekennzeichnet, dass** die zweite Klaue (Pb) eine Schwenkklaue ist, die relativ zu einer Schwenkachse um 360° drehbar ist und eine Wahl ermöglicht zwischen:
einer ersten Fläche der zweiten Klaue (Pb), die eine rauhe Oberfläche und eine zylindrische Ausnehmung aufweist, welche in der Lage ist, eine Osteosynthese-Zwischenscheibe gegen den Knochen zu drücken,
und einer zweiten Fläche der zweiten Klaue (Pb), die eine Ausnehmung mit Flanschen an den Rändern aufweist, welche in der Lage sind, eine Osteosynthese-Platte gegen den Knochen zu drücken.

2. Vorrichtung zur Fixierung von Schrauben in osteoporotischen Knochen nach dem vorherigen Anspruch, **dadurch gekennzeichnet, dass** sie einen Blockiermechanismus aufweist, wobei der Blockiermechanismus einen Druckknopf, ein elastisches Blockierelement und eine Zahnstange in Form von Sägezähnen aufweist.

3. Vorrichtung zur Fixierung von Schrauben in osteoporotischen Knochen nach einem der vorherigen Ansprüche, **dadurch gekennzeichnet, dass** die erste Klaue (Pa) eine Schwenkklaue ist, die relativ zu einer Schwenkachse drehbar ist und eine Wahl ermöglicht zwischen:
einer ersten Fläche der ersten Klaue (Pa), die eine Ausnehmung mit geometrischer Form aufweist, welche zur Aufnahme des biokompatiblen Fixierelements in der Lage ist;
und einer zweiten Fläche der ersten Klaue (Pa), wobei die zweite Fläche der ersten Klaue (Pa) gerändelt ist.

4. Vorrichtung zur Fixierung von Schrauben in osteoporotischen Knochen nach einem der vorherigen Ansprüche, **dadurch gekennzeichnet, dass** die zweite Fläche der zweiten Klaue (Pb) einen zylindrischen Teil mit einem ovalen Durchgangsloch und mit zwei Flanschen an den Endrändern des Durchgangslochs aufweist.

5. Vorrichtung zur Fixierung von Schrauben in osteoporotischen Knochen nach einem der vorherigen Ansprüche, **dadurch gekennzeichnet, dass** der männliche Teil (A) eine prismatische Stange (A3) mit einer Teilungsskala aufweist und dass der weibliche Teil (B) ein Gleitteil aufweist, wobei die Teilungsskala und das Gleitteil in der Lage sind, die Öffnung der ersten Klaue (Pa) und der zweiten Klaue (Pb) zu messen.

6. Vorrichtung zur Fixierung von Schrauben in osteoporotischen Knochen nach einem der vorherigen Ansprüche, **dadurch gekennzeichnet, dass** die Längsachsen der Griffe (ma, mb) in der gleichen vertikalen Ebene verlaufen wie die Längsachsen des ersten Arms (A1) und des zweiten Armes (B1) und dass die Griffe (ma, mb) einen Öffnungsbereich bilden, der in Gegenrichtung zu dem Öffnungsbereich weist, welcher von dem ersten Arm (A1) und dem zweiten Arm (B1) gebildet ist.

7. Vorrichtung zur Fixierung von Schrauben in osteoporotischen Knochen nach Anspruch 5 oder Anspruch 6 in Abhängigkeit von Anspruch 5, **dadurch gekennzeichnet, dass** die Vorrichtung ein Schließelement (C) an einem Ende der prismatischen Stange (A3) des männlichen Teils (A) aufweist, wobei das Schließelement (C) in der Lage ist, den Öffnungsgrad der ersten Klaue (Pa) und der zweiten Klaue (Pb) zu begrenzen, und wobei das Schließelement (C) aufweist:
eine äußere zylindrische Form;
ein Sackloch mit prismatischem Querschnitt, der an eine Form des Endes der prismatischen Stange anpassbar ist;
und eine gemäß dem unverlierbaren Typ ausgebildete Rückhalteschraube mit einem konischen Ende, die rechtwinklig in eine Seitenfläche des Schließelements (C) schraubbar und in eine in die prismatische Stange gebohrte Öffnung einführbar ist.

8. Vorrichtung zur Fixierung von Schrauben in osteoporotischen Knochen nach einem der vorherigen Ansprüche, **dadurch gekennzeichnet, dass** der Konnektor (cg) mit austauschbaren Bormeißel-Führungen (g) mehrerer Durchmesser verbindbar ist.

9. Vorrichtung zur Fixierung von Schrauben in osteoporotischen Knochen nach Anspruch 5 oder einem der vorherigen Ansprüche in Abhängigkeit von Anspruch 5, **dadurch gekennzeichnet, dass** die Vorrichtung eine Druckfeder (R) aufweist, die koaxial mit der prismatischen Stange (A3) des männlichen Teils (A) montiert ist, wobei die Druckfeder in der Lage ist, die Griffe (ma, mb) des männlichen Teils (A) und des weiblichen Teils (B) getrennt zu halten.

10. Vorrichtung zur Fixierung von Schrauben in osteoporotischen Knochen nach einem der vorherigen Ansprüche, **dadurch gekennzeichnet, dass** die Vorrichtung einen Druckbolzen aufweist, der in der Lage ist, eine Relativbewegung zwischen dem männlichen Teil (A) und dem weiblichen Teil (B) zu sperren und zu entsperren, wobei der Druckbolzen einen Kopf aufweist, mittels dessen der Druckbolzen durch manuelles Aufbringen eines Festzieh-Drehmoments verschraubt werden kann.

11. Vorrichtung zur Fixierung von Schrauben in osteoporotischen Knochen nach einem der vorherigen Ansprüche, **dadurch gekennzeichnet, dass** das biokompatible Fixierelement ein laminarförmiges Fixierelement mit einer Perforation ist, wobei die Perforation einen Durchmesser hat, der kleiner als ein Gewinde der Schraube ist.

12. Vorrichtung zur Fixierung von Schrauben in osteoporotischen Knochen nach einem der vorherigen Ansprüche, **dadurch gekennzeichnet, dass** die Oberflächen des biokompatiblen Fixierelements an Eigenschaften einer Knochen-Cortex angepasst sind, an der die Fixierelemente platzierbar sind.

## Revendications

1. Dispositif pour fixer une vis dans un os ostéoporotique comprenant :
un élément de fixation biocompatible (1) et un instrument facilitant sa mise en place, l'instrument comprenant :
une partie mâle (A) et une partie femelle (B), la partie mâle (A) et la partie femelle (B) ayant une surface de contact entre elles et un degré de liberté de mouvement relatif ; la partie mâle (A) et la partie femelle (B) comprenant des poignées (ma, mb) et la partie mâle (A) et la partie femelle (B) étant adaptées pour coulisser de manière linéaire l'une par rapport à l'autre ;
une première mâchoire (Pa) assemblée dans un premier bras en angle droit (A1) formant une extrémité de la partie mâle (A) à déplacer à volonté, la première mâchoire (Pa) étant adaptée pour loger et transporter l'élément de fixation biocompatible (1) ;
une seconde mâchoire (Pb) assemblée dans un second bras en angle droit (B1) formant une extrémité de la partie femelle (B) ;
et un élément de guidage (g) raccordé au second bras en angle droit (B1) au moyen d'un connecteur (cg), une vis pouvant être introduite à travers l'élément de guidage (g) ;
**caractérisé en ce que** la seconde mâchoire (Pb) est une mâchoire pivotante adaptée pour tourner à 360° par rapport à un axe de pivot et permet de sélectionner entre :
une première face de la seconde mâchoire (Pb) comprenant une surface rugueuse et un creux cylindrique adapté pour comprimer une rondelle pour ostéosynthèse contre l'os ; et
une seconde face de la seconde mâchoire (Pb) comprenant un creux avec des brides dans les bords adaptés pour comprimer une plaque pour ostéosynthèse contre l'os.

2. Dispositif pour fixer des vis dans des os ostéoporotiques selon la revendication précédente, **caractérisé en ce qu'**il comprend un mécanisme formant bloc, le mécanisme formant bloc comprenant un bouton poussoir, un élément de blocage élastique et une crémaillère en forme de dents de scie.

3. Dispositif pour fixer des vis dans des os ostéoporotiques selon l'une quelconque des revendications précédentes, **caractérisé en ce que** la première mâchoire (Pa) est une mâchoire pivotante adaptée pour tourner par rapport à un axe de pivot, permettant de sélectionner entre :
une première face de la première mâchoire (Pa) comprenant un creux avec une forme géométrique adaptée pour loger l'élément de fixation biocompatible ;
et une seconde face de la première mâchoire (Pa), la seconde face de la première mâchoire (Pa) étant moletée.

4. Dispositif pour fixer des vis dans des os ostéoporotiques selon l'une quelconque des revendications précédentes, **caractérisé en ce que** la seconde face de la seconde mâchoire (Pb) comprend une partie cylindrique avec un trou de passage ovale central et deux brides dans les bords d'extrémité du trou de passage.

5. Dispositif pour fixer des vis dans des os ostéoporotiques selon l'une quelconque des revendications précédentes, **caractérisé en ce que** la partie mâle (A) comprend une barre prismatique (A3) avec des échelles graduées et **en ce que** la partie femelle (B) comprend un curseur, les échelles graduées et le curseur étant adaptés pour mesurer l'ouverture de la première mâchoire (Pa) et de la seconde mâchoire (Pb).

6. Dispositif pour fixer des vis dans des os ostéoporotiques selon les revendications précédentes, **caractérisé en ce que** les axes longitudinaux des poignées (ma, mb) sont dans un même plan vertical en tant qu'axes longitudinaux du premier bras (A1) et du second bras (B1) ; et **en ce que** les poignées (ma, mb) forment une bouche orientée dans une direction opposée en tant que bouche formée par le premier bras (A1) et le second bras (B1).

7. Dispositif pour fixer des vis dans des os ostéoporotiques selon la revendication 5 ou la revendication 6 lorsqu'elle dépend de la revendication 5, **caractérisé en ce qu'**il comprend un élément de fermeture (C) dans une extrémité de la barre prismatique (A3) de la partie mâle (A), l'élément de fermeture (C) étant adapté pour limiter un degré d'ouverture de la première mâchoire (Pa) et de la seconde mâchoire (Pb) et l'élément de fermeture (C) comprenant :
une forme cylindrique externe ;
une perforation borgne avec une section prismatique adaptée pour être ajustée à une forme de l'extrémité de la barre prismatique ;
et une vis de retenue de type couplé avec une extrémité conique, adaptée pour être vissée perpendiculairement à une surface latérale de l'élément de fermeture (C) et pour entrer dans un orifice percé dans la barre prismatique.

8. Dispositif pour fixer des vis dans des os ostéoporotiques selon l'une quelconque des revendications précédentes, **caractérisé en ce que** le connecteur (cg) est adapté pour coupler des guide-forets (g) interchangeables avec une pluralité de diamètres.

9. Dispositif pour fixer des vis dans des os ostéoporotiques selon la revendication 5 ou l'une des revendications précédentes lorsqu'elle dépend de la revendication 5, **caractérisé en ce qu'**il comprend un ressort de compression (R) assemblé de manière coaxiale avec la barre prismatique (A3) de la partie mâle (A), adapté pour maintenir les poignées (ma, mb) de la partie mâle (A) et de la partie femelle (B) séparées.

10. Dispositif pour fixer des vis dans des os ostéoporotiques selon l'une quelconque des revendications précédentes, **caractérisé en ce qu'**il comprend un boulon de pression adapté pour bloquer et débloquer un mouvement relatif entre la partie mâle (A) et la partie femelle (B), le boulon de pression comprenant une tête adaptée pour visser le boulon de pression en appliquant manuellement un couple de serrage.

11. Dispositif pour fixer des vis dans des os ostéoporotiques selon l'une quelconque des revendications précédentes, **caractérisé en ce que** l'élément de fixation biocompatible est un élément de fixation de forme laminaire avec une perforation, la perforation ayant un diamètre plus petit qu'un filetage de la vis.

12. Dispositif pour fixer des vis dans des os ostéoporotiques selon l'une quelconque des revendications précédentes, **caractérisé en ce que** les surfaces de l'élément de fixation biocompatible sont adaptées aux caractéristiques d'un cortex osseux pour lequel sont adaptés les éléments de fixation destinés à y être placés.
